# EUROPEAN PATENT SPECIFICATION

(11) **EP 4 239 576 B1**
(45) Date of publication and mention of the grant of the patent: **01.04.2026**
(21) Application number: 22160167.7
(22) Date of filing: 04.03.2022
(51) Int. Cl.: G06T 7/30, A61B 8/00, A61B 8/08

(54) **METHOD AND SYSTEM FOR REGISTERING IMAGES ACQUIRED WITH DIFFERENT MODALITIES FOR GENERATING FUSION IMAGES FROM REGISTERED IMAGES ACQUIRED WITH DIFFERENT MODALITIES**
VERFAHREN UND SYSTEM ZUR REGISTRIERUNG VON MIT VERSCHIEDENEN MODALITÄTEN AUFGENOMMENEN BILDERN ZUR ERZEUGUNG VON FUSIONSBILDERN AUS REGISTRIERTEN, MIT VERSCHIEDENEN MODALITÄTEN AUFGENOMMENEN BILDERN
PROCÉDÉ ET SYSTÈME DE RECALAGE D'IMAGES ACQUISES AVEC DIFFÉRENTES MODALITÉS POUR GÉNÉRER DES IMAGES DE FUSION À PARTIR D'IMAGES RECALÉES ACQUISES AVEC DIFFÉRENTES MODALITÉS

(43) Date of publication of application: 06.09.2023
(73) Proprietor: Esaote S.p.A., 16152 Genova (IT)
(72) Inventor: DE BENI, STEFANO, 16123 GENOVA (IT); CROCCO, MARCO, 15076 OVADA (AL) (IT); ZINI, LUCA, 16147 GENOVA (IT); TRAVERSINI, SARA, 16010 SAVIGNONE (GE) (IT)
(74) Representative: Karaghiosoff, Giorgio Alessandro

(56) References cited:
- US-B2- 10 675 006
- US-B2- 10 726 555
- FU YABO ET AL: "Deep learning in medical image registration: a review", PHYSICS IN MEDICINE & BIOLOGY, vol. 65, no. 20, 24 December 2020 (2020-12-24), pages 1 - 48, XP055886695, DOI: 10.1088/1361-6560/ab843e
- ELMAHDY MOHAMED S ET AL: "Adversarial Optimization for Joint Registration and Segmentation in Prostate CT Radiotherapy", 10 October 2019, ARXIV.ORG, CORNELL UNIVERSITY LIBRARY, 201 OLIN LIBRARY CORNELL UNIVERSITY ITHACA, NY 14853, PAGE(S) 366 - 374, XP047522357

## Description

### Background of the Invention

In the field of medical imaging different image acquisition techniques are currently in use. Each technique allows to obtain image data information related to different kind of tissues and with different image features such as signal to noise ratio, contrast, resolution, detection of different tissue kinds, and others.

Generally imaging techniques furnishing high resolution image data have the drawback of requesting more or less long image acquisition times, so that this imaging techniques are not eligible when real time images are needed. This is for example the case when the images are needed for the monitoring of a surgical tool such as for example for monitoring and guiding the insertion of a biopsy needle, or when functional reactions of an organ or a tissue are to be monitored which are related to rapid status change, such, as for example cardiological imaging related to the monitoring of the heart functions.

Typical high resolution imaging techniques can be for example but not exhaustively CT-imaging, PET-imaging, MRI, or other imaging techniques.

Ultrasound imaging has the advantage to allow to acquire real time images of a target region, but have a far reduced field of view than the other imaging techniques and furthermore ultrasound imaging have a lower quality in relation to features, such as particularly image resolution and or the revealing and reproducing in the image of certain tissue kinds, such as for example soft tissues.

In carrying out medical imaging diagnostics is therefore a current practice to combine real time ultrasound images and images acquired by one further image acquisition modality. It is also known to combine real time ultrasound images with images acquired by more than one further image acquisition modality.

This kind of image combination or fusion is used in several applications as for example in relation to organs carrying out displacements by it own motion, and/or when considering hematic fluxes or in monitoring and tracking interventional tools, such as laparoscopic surgical tools and/or needles, in real time during intervention.

The one or more further imaging modalities which images are combined with the real time ultrasound images can be imaging modalities of the radiological kind, this meaning imaging modalities which allow to acquire images of the internal anatomy, such as CT-imaging, MRI or PET-imaging or similar or may also be imaging modalities which are of the optical kind directed to combining optical images of a target body related to the external anatomy with internal images of the target body.

The known systems and methods all requests the registering of the real time ultrasound images with the images acquired with the one or more further imaging method. Registering is carried out by tracking the position, orientation and the displacements of the ultrasound probe during real time ultrasound image acquisition within a reference system which is common both to the ultrasound images and to the images acquired with the one or more further imaging modes. This implies the use of probe tracker. These probe trackers are relatively expensive devices with dedicated accessories that complicate the normal workflow.

In order to avoid the use of probe trackers for registration, alternative registration methods are known making use of artificial intelligence and particularly of Machine Learning algorithms.

Image registration is the process of interpreting several images to a common coordinate system. Registration may be used to place every image acquired into a common frame of reference so that the images acquired with different imaging modalities may be used to generate a combined image having higher information content.

Several alternatives are known and the following list is a non-exhaustive list of alternatives known in the art:
One-shot registration of heterogeneous modalities by ML-algorithms(Machine Learning algorithms)
mapping by Machine Learning algorithms of MRI/CT image to "synthetic" ultrasound data and subsequent registration to real ultrasound image
Segmentation by Machine Learning algorithms of anatomical structure in ultrasound images and registration with previously segmented MRI/CT data.

Document US10726555B2, for example, discloses Joint registration and segmentation of images using deep learning. In this document, A system for registering and segmenting images includes an image scanner configured to acquire an image pair including a first image at a first time and a second image at a second time that is after the first time. A joint registration and segmentation server receives the image pair from the image scanner and simultaneously performs joint registration and segmentation on the image pair using a single deep learning framework. A computer vision processor receives an output of the joint registration and segmentation server and characterizes how a condition has progressed from the first time to the second time therefrom. A user terminal presents the characterization to a user. Joint registration and segmentation on the set of images is performed using the constructed neural network or other DL model/classifier. A cost function may be defined for registering an image pair based on intensity information and segmentation information. Generative adversarial networks (GANs) may be used to register an image pair. The GANs may take, as input, the training data, which may include pairs of images including a reference image and a floating image. The floating image may then be registered to, or otherwise aligned to, the reference image by transforming the floating image appropriately. A segmentation mask of the reference image may be generated either manually or using a deep learning (DL) based segmentation method. Then, using the reference image, the floating image, and the segmentation mask, the GAN of the registration network outputs the registered image which is a transformed version of the floating image. Simultaneously, the network may also output a segmentation mask of the transformed image.

Data processing of medical images by a generative adversarial network is disclosed in document US10592779B2. Here the GAN are used for training a classifier for processing medical images.

Although these methods and systems provide for tracker-less registration of multimodal images, i.e. images acquired with different imaging methods, the above systems and methods requires that large, panoramic real time ultrasound images this meaning ultrasound images having high depth and/or large FOV (field of view) are acquired to achieve the required precision while current real time ultrasound images are often zoomed and at shallow depth, especially in obstetric or muscle-skeletal applications.

The need of acquiring and reconstructing ultrasound panoramic images is in contrast to the acquisition of real time images and requires high computational power of the ultrasound system.

In the technical field it is known to generate images on a display or screen of a monitor by using a so-called interlaced scan. Interlaced scan is a technique for doubling the perceived frame rate of a video display without consuming extra bandwidth. The interlaced signal contains two fields of a video frame captured consecutively. This enhances motion perception to the viewer and reduces flicker. Images related to video frames are generated by scanning or displaying each line of the image corresponding to a row of pixels of an array of pixels forming the image. According to the interlaced scan mode two fields are used to create a frame one field contains all odd numbered lines, i.e. the odd-numbered rows of pixels forming the image and the other field contains every even-numbered lines, i.e. every even-numbered rows of pixels. For example, the interlaced scan pattern in a standard definition CRT display completes a scan in two passes (two fields). The first pass displays the first and all odd numbered lines, from the top left corner to the bottom right corner. The second pass displays the second and all even numbered lines, filling in the gaps in the first scan.

US10675006B2 discloses a method of multi-modality imaging fusion with ultrasound, in which one transducer is used for registering ultrasound scan data with scan data from another mode. This registration is used to then align scan data from a different ultrasound transducer with the scan data of the other mode. The alignment may account for differences in position sensing between the two transducers.

### Summary of the invention

A first object of the present invention relates to providing an improved tracker-less method and system for registering images acquired with different modalities for generating fusion images from registered images acquired with different modalities. The improvement consisting in overcoming the above-described draw backs of the current method and system.

According to a first embodiment a tracker-less, processor-implemented method for registering images acquired with different modalities for generating fusion images from registered images acquired with different modalities, the said method comprising the following steps:
Acquiring ultrasound images by interlacing a wide, high depth ultrasound scan to a zoomed ultrasound scan;
Registering the image data obtained from the said high-depth ultrasound scan with image data of the same anatomical region acquired with a different modality and determining registration data;
The said image data obtained from the said high-depth ultrasound scan and/or the said image data acquired with the different modality not being displayed to the user;
Registering the said image data acquired by the said zoomed ultrasound scan with corresponding zoomed image data obtained with the said different modality by applying the said registration data to the image data acquired by the said zoomed ultrasound scan;
Combining and/or fusing the said registered image data acquired by the said zoomed ultrasound scan with the zoomed image data obtained with the said different modality and
Displaying the said combined or fused image data acquired by the said zoomed ultrasound scan with the zoomed image data obtained with the said different modality.

According to an embodiment registration is carried out by means of registration algorithms comprising the steps of:

Defining landmarks on the image acquired by the different modality and by the wide, high-depth ultrasound scan;
defining a spatial reference system common to both said images;
determining transfer functions of the image pixels of the image according to the different modality to the image pixels of the image acquired by the high-depth ultrasound scan based on the different spatial positions of the said landmarks in the common reference system.

In order to register the image acquired by the high-depth ultrasound scan with the image acquired with the different imaging modality the said transfer functions are applied to the image pixels of the said image acquired by the high-depth ultrasound scan.

In relation to the above embodiments the transfer functions, also called registration data, are applied to the image pixels obtained by the zoomed ultrasound scan which is combined with a correspondingly zoomed field of view of the image acquired by the different modality and only the said combined image is displayed to the user.

Many different landmarks may be chosen depending on the target anatomical district to be imaged. Some landmarks are typical anatomical features of the imaged target.

Alternatively, or in combination, one or more landmarks can be in the form of added elements which have specific and univocally associated features.

Registration algorithm can be in the form of cross correlation algorithm or may be inspired by so called optical flow technologies such as for example the one disclosed in EP05425048.

According to a further embodiment, the registration can be carried out by a method comprising the following steps:
a) Providing at least a first and a second digital or digitalized image or set of cross-sectional images of the same object, the said images being formed by a two or three dimensional array of pixels or voxels;
b) Defining within the first image or set of images a certain number of landmarks, so called features by selecting a certain number of pixels or voxels which are set as landmarks or features and generating a list of said features to be tracked;
c) Tracking the position of each pixel or voxel selected as a feature from the first to a second or to an image or set of images acquired at later time instants by determining the optical flow vector between the positions from the first to the said second image or to the said image or set of images acquired at later time instants for each pixel or voxel selected as a feature;
d) Registering the first and the second image or the image or the set of images acquired at later times by applying the inverse optical flow vector to the position of pixels or voxels of the second image or set of images,
wherein an automatic trackable landmark selection step is carried out, comprising the following steps:
B1) defining a pixel or voxel neighborhood window around each pixel or voxel of the first image or first set of cross-sectional images, the said pixel or voxel neighborhood window comprising a limited number of pixels or voxels;
B2) for each target pixel or voxel determining a set of two or more characteristic parameters which are calculated as a function of the numeric parameters describing the appearance, so called numeric appearance parameters, of the said target pixel or voxel and of each or a part of the pixels or voxels of the said pixel or voxel window and as a function of one or more characteristic parameters of either the matrix of numeric parameters representing the pixels or voxels of the said window or of a transformation of the said matrix of numeric parameters;
B3) determining the pixels or voxels consisting in validly trackable landmark or feature as a function of the said characteristic parameters of the target pixels or voxels, characterized in that the set of characteristic parameters is subdivided in a certain number of subsets of characteristic parameters and for each subset a secondary characteristic parameter is determined as a function of the characteristic parameter of the said subset, a threshold being defined for each of the secondary characteristic parameter and for each secondary characteristic parameter, the quality of validly trackable and non validly trackable landmark being determined by a function of the said secondary characteristic parameter and the corresponding threshold.

This embodiment of registration method is disclosed with details in EP1941453

A specific variant embodiment disclosed in EP1941453 provides for a method in which the quality of validly trackable and non validly trackable landmark of a target pixel or voxel is determined by processing the secondary characteristic parameters of each pixel or voxel of the image with a machine learning algorithm and specifically a classification algorithm.

According to this specific embodiment the said method comprises the following steps:
providing a certain number of images of known cases in which a certain number of valid landmarks has been identified as validly trackable landmarks or features;
Determining the set of characteristic parameters for the pixels or voxels corresponding to the said landmarks identified validly trackable in the certain number of images by applying the automatic trackable landmark selection step consisting in the steps B1) and B2) disclosed above;
Generating a vector univoquely associated to each landmark identified as validly trackable and comprising as components the said characteristic parameters of the pixels or voxels coinciding with the validly trackable landmarks;
Describing the quality of validly trackable landmark by means of a predetermined numerical value of a variable and associating the said numerical value to the vector coding each pixel or voxel coinciding with a validly trackable landmark;
Each vector coding each pixel or voxel coinciding with a validly trackable landmark forming a record of a training database for a classification algorithm;
Training a classification algorithm by means of the said database;
Determining the quality of validly trackable landmark of a target pixel or voxel by furnishing to the input of the trained classification algorithm the vector comprising the characteristic parameters of the said target pixel or voxel.

Currently new machine learning algorithms so called generative algorithm has been developed which provides for the generation of registered image data in relation to a reference image such as a first image as for example a first image acquired by a first imaging modality.

According to an embodiment registration data may be determined by using a so called generative algorithm as the so called GAN.

An example of a GAN algorithm used for determining registration data is disclosed in Document US10726555B2 which has been discussed with further details in the preceding description.

In using Machine Learning algorithm in order to generate tracker-less registration data of an ultrasound image in relation to an image previously acquired with another imaging modality or even optionally with the same imaging modality the machine learning algorithm may be applied according to different alternatives.

According to a first alternative, a one-shot machine learning registration of heterogeneous imaging modalities may be applied.

According to a further alternative the Machine Learning algorithm may be used for mapping the image acquired by a different modality such as for example MRI or CT to a "synthetic" ultrasound image subsequent registration to a real ultrasound image.

According to a third alternative, a Machine Learning algorithm may be used for carrying out an anatomical segmentation in the ultrasound images and the registration with previously segmented images acquired by the other modality (such as MRI or CT for example).

The invention relates also to a system which is configured for registering images acquired with different modalities for generating fusion images from registered images acquired with different modalities according to the combination of features of independent claim 7.

According to an embodiment, the processing unit as well as the image combination unit can be in the form of a software coding the instructions for a processing unit to carry out the above disclosed functions.

In a variant embodiment the said software may be loaded and executed by a processing unit which is integrated or part of a CPU of the ultrasound system.

In a variant embodiment the said software may be loaded and executed by an external CPU which is communicating with the controller of the ultrasound system and with the display of the ultrasound system.

A further Variant embodiment provides that part of the software is loaded and executed by the processing unit, which is integrated, or part of a CPU of the ultrasound system and part of the software is loaded and executed by said external CPU.

Further embodiments of the method and of the system are described in the following description and are subject of the dependent claims.

### BRIEF DESCRIPTION OF THE DRAWINGS

Figure 1 is a high level block diagram of an embodiment of the system according to the present invention in which the processing unit the registration processor and the image combination unit are integrated as software tools in an ultrasound system.
Fig 2 is a diagram illustrating the steps of the method according to the present invention.
Fig. 3 is a diagram illustrating a high level functional diagram of a Generative Adversarial Network configured for carrying out the registration according to an embodiment of the present invention.
Fig. 4 to fig. 6 are diagrams illustrating a more specific possible Generative Adversarial Network and two alternative discriminators operating with a specific loss function.
Figure 7 to 9 show the diagrams of an alternative embodiment of an ultrasound system which is configured by loading and executing specific software the functions of a method and a system according to the present invention.

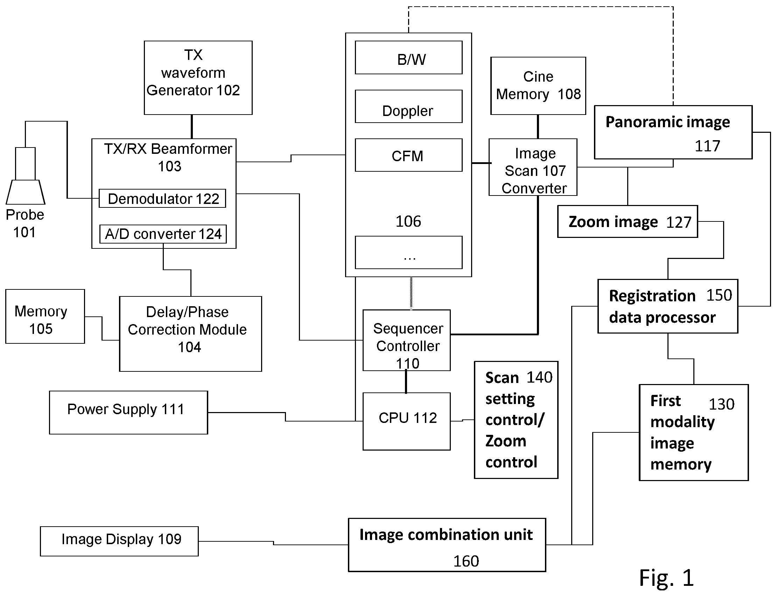

Fig. 1 illustrates a high-level block diagram of an ultrasound system implemented in accordance with embodiments herein. Portions of the system (as defined by various functional blocks) may be implemented with dedicated hardware, analog and/or digital circuitry, and/or one or more processors operating program instructions stored in memory. Additionally or alternatively, all or portions of the system may be implemented utilizing digital components, digital signal processors (DSPs) and/or field programmable gate arrays (FPGAs) and the like. The blocks/modules illustrated in Fig. 1 can be implemented with dedicated hardware (DPSs, FPGAs, memories) and/or in software with one or more processors.

The ultrasound system of Fig. 1 includes one or more ultrasound probes 101. The probe 101 may include various transducer array configurations, such as a one dimensional array, a two dimensional array, a linear array, a convex array and the like. The transducers of the array may be managed to operate as a 1D array, 1.25D array, 1.5D array, 1.75D array, 2D array, 3D array, 4D array, etc.

The ultrasound probe 101 is coupled over a wired or wireless link to a beamformer 103. The beamformer 103 includes a transmit (TX) beamformer and a receive (RX) beamformer that are jointly represented by TX/RX beamformer 103. The TX and RX portions of the beamformer may be implemented together or separately. The beamformer 103 supplies transmit signals to the probe 101 and performs beamforming of "echo" receive signals that are received by the probe 101.

A TX waveform generator 102 is coupled to the beamformer 103 and generates the transmit signals that are supplied from the beamformer 103 to the probe 101. The transmit signals may represent various types of ultrasound TX signals such as used in connection with B-mode imaging, Doppler imaging, color Doppler imaging, pulse-inversion transmit techniques, contrast-based imaging, M-mode imaging and the like. Additionally, or alternatively, the transmit signals may include single or multi-line transmit, shear wave transmit signals and the like.

The beamformer 103 performs beamforming upon received echo signals to form beamformed echo signals in connection pixel locations distributed across the region of interest. For example, in accordance with certain embodiments, the transducer elements generate raw analog receive signals that are supplied to the beamformer. The beamformer adjusts the delays to focus the receive signal along one or more select receive beams and at one or more select depths within the region of interest (ROI). The beamformer adjusts the weighting of the receive signals to obtain a desired apodization and profile. The beamformer applies weights and delays to the receive signals from individual corresponding transducers of the probe. The delayed, weighted receive signals are then summed to form a coherent receive signa.

The beamformer 103 includes (or is coupled to) an A/D converter 124 that digitizes the receive signals at a select sampling rate. The digitization process may be performed before or after the summing operation that produces the coherent receive signals. The beamformer also includes (or is coupled to) a demodulator 122 that demodulates the receive signals to remove the carrier waveform. The demodulation may be performed before or after the summing operation. Once the receive signals are demodulated and digitized, complex receive signals are generated that include I,Q components (also referred to as I,Q data pairs). The I,Q data pairs are saved as image pixels in memory. The I,Q data pairs, defining the image pixels for corresponding individual locations along corresponding lines of sight (LOS) or view lines. A collection of image pixels (e.g., I,Q data pairs) are collected over time and saved as 2D image frames and/or 3D volumes of image data. The image pixels correspond to tissue and other anatomy within the ROI.

A dedicated sequencer/timing controller 110 may be programmed to manage acquisition timing which can be generalized as a sequence of firings aimed at selecting reflection points/targets in the ROI. The sequence controller 110 manages operation of the TX/RX beamformer 103 in connection with transmitting ultrasound beams and measuring image pixels at individual LOS locations along the lines of sight. The sequence controller 110 also manages collection of receive signals.

One or more processors 106 perform various processing operations as described herein.

In accordance with embodiments herein, the beamformer 103 includes an input that configured to be coupled to an ultrasound probe 101 and receive signals from transducers of the ultrasound probe 101. The demodulator 122 demodulates the receive signals to generate complex receive signals by removing the carrier from the receive signal. The memory 105 stores time delays to align contributions of reflection signals received by the transducers of the array of the probe 101. The memory 105 also stores phase corrections to correct phase differences introduced by the time delays.

A delay/phase correction (DPC) module 104 is coupled to the memory 105 and provides various delays and corrections (e.g., coarse, fine, etc.) to the beamformer 103. For example, the DPC module 104 directs the beamformer 103 to apply time delay and phase correction to the complex receive signals to form delayed complex receive signals. The beamformer 103 then sums, in a coherent manner, the delayed complex received signals to obtain a coherent receive signal in connection with a reflection point or a reflection target.

A memory 105 may store coarse corrections calculated as a multiple of a sampling time. A common coarse correction may be stored in connection with multiple channels. Alternatively, different coarse corrections may be stored in connection with various corresponding channels. The memory 105 may also store fine corrections calculated as a fraction of the sampling time. Different fine corrections are be stored in connection with various corresponding channels based on the calculations described herein. As explained herein, the beamformer 103 (circuitry) is configured to apply the coarse and fine corrections contemporaneously by multiplying the complex receive signals by a complex carrier delayed by the multiple of the sampling time and by the fraction of the sampling time.

The memory 105 may store a pre-calculated table, where the pre-calculated table comprises real times of arrival of the receive signals relative to a predetermined reflection point. Optionally, the processor 106 may be configured to calculate real times of arrival of the receive signals relative to a predetermined reflection point. The processor 106 may be configured to calculate the coarse delay for baseband signal components of the complex receive signals, in connection with a plurality of channels, by a round function of real times of arrival associated with each of the channels. The processor 106 may be configured to calculate a fractional value of the fine correction based on real times of arrival for a plurality of channels.

The beamformer 103 circuitry may further comprise a complex multiplier configured to multiply the fractional value by the complex receive signal for the corresponding channel to which the corresponding coarse correction has been added.

In accordance with certain embodiments, at least a portion of the beamforming process may be implemented by the processor 106 (e.g., in connection with software-based beamforming). For example, the memory 105 may store beamforming related program instructions that are implemented by the processor 106 to apply fine and coarse corrections to the complex receive signals.

The processor 106 may be configured to provide parallel multi-line receive (PMR) fine correction in baseband in connection with individual view lines acquired in parallel contemporaneously with a focusing function.

The processor 106 and/or CPU 112 also performs conventional ultrasound operations. For example, the processor 106 executes a B/W module to generate B-mode images. The processor 106 and/or CPU 112 executes a Doppler module to generate Doppler images. The processor executes a Color flow module (CFM) to generate color flow images. The processor 106 and/or CPU 112 may implement additional ultrasound imaging and measurement operations. Optionally, the processor 106 and/or CPU 112 may filter the first and second displacements to eliminate movement-related artifacts.

The processor and/or CPU 112 may be also configured to setup the transmitted and/or received ultrasound beams in order to cover alternatively a high-depth and large field of view scan of the target region and a zoomed scan of a limited zone of the said target region. The settings may be saved in the memory 105. The sequence controller 110 may be configured or driven to control the ultrasound system for carrying out in an interlaced manner an ultrasound scan for acquiring a high-depth and large FoV (Field of View) image and a zoomed image as defined above and according to the setup of the parameters settings for the beamformers saved in the memory 105.

In the present exemplary embodiment, a setting input interface indicated by 140 is provided which allows the user to define the parameters for acquiring the high-depth and large FoV image and/or the zoomed image.

An image scan converter 107 performs scan conversion on the image pixels to convert the format of the image pixels from the coordinate system of the ultrasound acquisition signal path (e.g., the beamformer, etc.) and the coordinate system of the display. For example, the scan converter 107 may convert the image pixels from polar coordinates to Cartesian coordinates for image frames.

As it is indicated with more details in figure 1, in accordance with the interlaced execution of the high-depth and large FoV scans and the zoomed scans the scan converter 107 generates two images a panoramic image 117 corresponding to the image acquired by the high-depth and large Fov ultrasound scans and a zoomed image 127 corresponding to the zoomed ultrasound scans.

According to a variant embodiment the Panoramic image 117 may not be processed by the scan converter and be maintained in the form of the image data provided by the processor 106 according to one or more of the imaging modes indicated such as B-mode, Doppler-mode, etc..

A processing unit, indicated as a separate registration processor 150, is configured for executing a registration process of the panoramic image 117 with the first modality image saved in memory 130. In this case the panoramic image and/or the first modality images are not displayed on the display 109.

Registration process may be executed by one or more of the different processing algorithms disclosed in the previous description such as, for example,
using the definition of registration data, such as transfer functions of the image pixels of one image into the reference system of the other image which are calculated by means of realignment of landmarks provided on the panoramic image 117 and the first modality image by cross correlation, optical flow methods and other registration algorithms which operates in defining transfer functions or optical flow functions for translation and or rotation of pixels or groups of pixels;
using machine learning algorithms which are trained in order to provide registration data by generating "synthetic registered images" with the said first modality image.

Different kinds of Machine Learning algorithms may be used according to the disclosure of the previous description and of some specific exemplary embodiments disclosed in the following.

A memory 130 is provided in which an image according to a first imaging modality is saved. The first modality image saved in memory 130 may be converted in a format corresponding to the one of the panoramic images 117.

Although the registration processor 150 is indicated as a separate processing unit, integrated in the boards of the ultrasound system, according to a first variant embodiment, the hardware of the said registration processor 150 may be, for example, the same CPU112 or the processor 106, or partly both processors 112 and 106. The functions of the registration processor 150 are in the form of a software program in which the instructions are coded and which, when executed by the CPU 112 and/or by the processor 106 configure the same one in such a way as to carry out the functions of the registration processor 150.

According to a variant embodiment the registration processor 150 and optionally also the memory 130 may be part of a separate processing device which communicates with the ultrasound system by communication interfaces such a wired communication interfaces, for example by any kind of possible networks and/or by a wireless communication interfaces such as tone or more of the currently available wireless communication protocols such as Wi-Fi-networks, Bluetooth, and other.

The registration data provided by the registration processor 150 are applied to the image obtained by the zoomed ultrasound scan in order to register the zoomed image with the corresponding zoomed region of the first modality image. An image combination unit 160 is configured to carry out image combination algorithms according to one or more different functions such as simple addition and/or convolution and/or fusion and other combination functions operating according to different criteria.

In relation to the Image combination unit 160, same variant embodiments are valid as for the registration processor 150. In one variant embodiment, a dedicated hardware is integrated in the boards of the ultrasound scanner. The said processing hardware is configured to carry out the image combination functions by one or more image combination algorithms and the instructions for carrying out the said one or more image combination algorithms are coded in the said image combination software. The image combination processor is thus configured to carry out the one or more image combination algorithms by executing the said software.

In a variant embodiment, the image combination processing hardware of the image combination unit is formed by the CPU 112 and/or the processor 106 and the image combination functions are carried out by the CPU112 and/or by the processor 106 or partly by the CPU112 and partly by the processor 106 by executing the said image combination software.

The third variant embodiment may provide that the said image combination unit is part of a separated image processing device which comprises also the registration processor 150 and optionally the memory 130 for the first image acquired by the image modality and the one or more communication interfaces with the ultrasound system as disclosed above.

The output of the Image combination unit 160 is then displayed on the display 109. This display is in the present embodiment the display of the ultrasound scanner but more displays may be provided at least one of which is a display of the separate image processing device or of a separate image displaying station which may be also provided at remote site relatively to the site at which the ultrasound system is located.

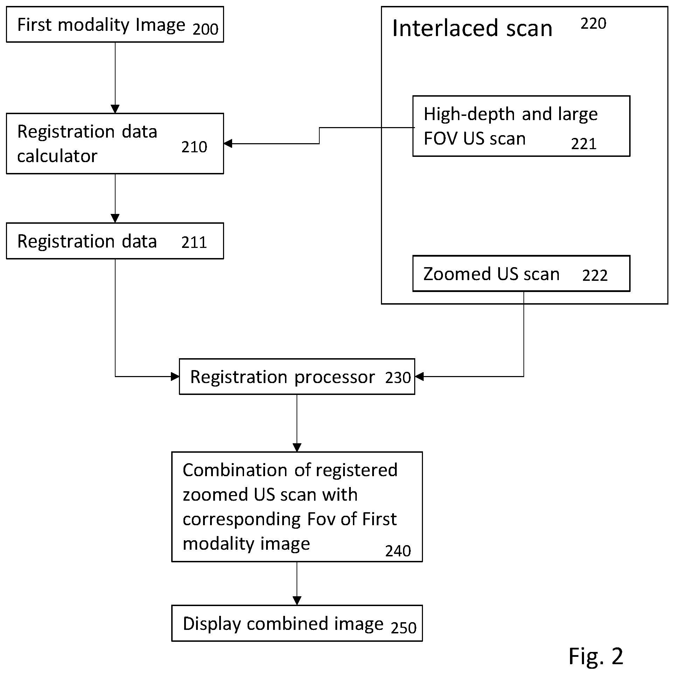

Figure 2 is a functional block diagram illustrating the steps of an embodiment of the present method.

As a first step 200 an image is acquired by means of a first imaging modality. Different imaging modalities may be chosen such as for example and non-exhaustively: MRI, CT, PET, Ultrasound.

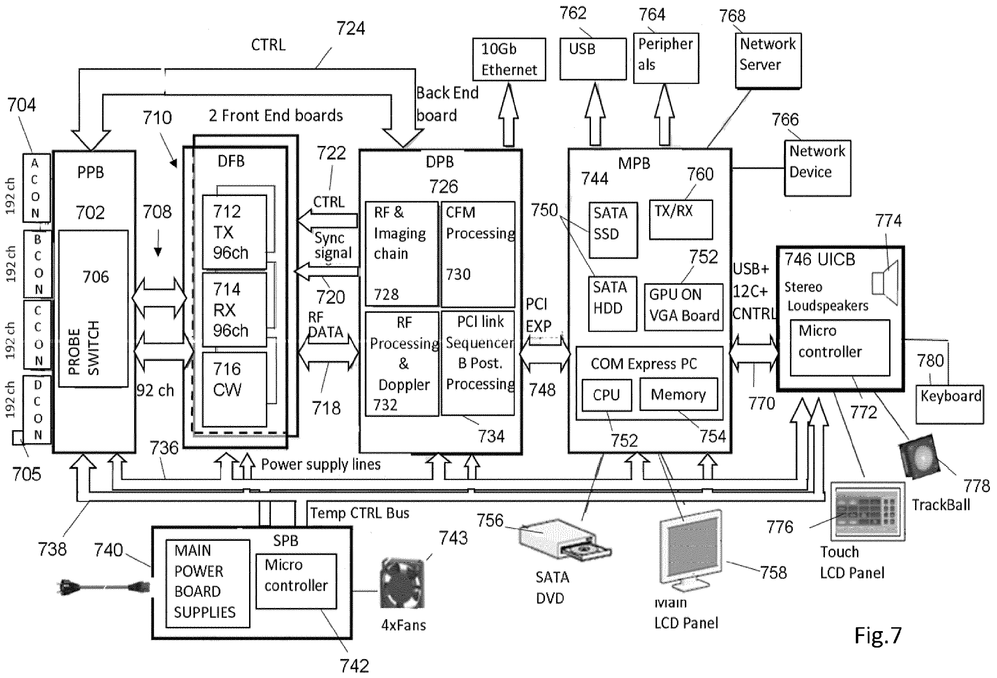

The said image acquired by the said first modality is stored in memory of a image processing system or device or of an imaging processing section of an ultrasound scanner, for example an ultrasound scanner as disclosed according to the embodiment of figure 1 and/or according to the embodiment of figures 7 to 9. In the present example the said memory is part of a processor which is configured to operate as a registration data calculator which is configured for carrying out the said registration data calculation according to step 210. At step 220 ultrasound scans of the same target body or target region are carried out for acquiring ultrasound images of the said target body or target region.

Ultrasound system is operated in such a way as to acquire in interlaced manner an ultrasound image by a high-depth and large Fov scan as indicated by 221 and an ultrasound image by a zoomed ultrasound scan as indicated by the step 222.

The High-depth and large FOV image data are not processed for display on the display screen of the ultrasound system or of the image processing device or on a remote scree, but are transmitted to the registration data calculator 210 and by using one or more of the different and alternative registration algorithm, and preferably but non exclusively a machine learning algorithm the registration data are determined from the image data relating to the high-depth and large Fov ultrasound scan 221 and from the image data according to the image acquisition with the first modality 200.

The registration data 211 and the image acquired by the ultrasound zoomed scan 222 are fed to a registration processor which apply the registration data to the said image acquired by the zoomed ultrasound scan 222. At step 240 the zoomed ultrasound scan image registered to the first modality image and the corresponding region of the first modality image are combined and the combined images are displayed at step 250. The display screen may be the one of the ultrasound scanner and/or the one of the device operating according to the said first imaging modality and/or a screen of a display associated to a separated system for carrying out the registration and the combination steps and/or any other screen at the same site of one of the said devices or at remote location.

As already disclosed in the preceding paragraphs many different methods can be applied for determining the registration data out of the panoramic ultrasound image 221 and the image acquired by the first modality 200. In one preferred embodiment which is not to be considered limiting, the said registration data are obtained by using a machine learning algorithm.

Many different machine learning algorithms are possible and recently a family of machine learning algorithms called Generative adversarial algorithms has bee developed. This kind of algorithms are able to generate synthetic images which represents real images deriving from some specific processing method, the said synthetic images not being determined by applying the said processing method but basing on a combination of predictive algorithms such as neural networks.

This kind of algorithms are able to be trained in a non-supervised manner and the training does not necessitate of too many training data records, so that these algorithms are preferred when the training data set is poor or is difficult or time consuming to be generated.

In a more generic definition Generative Adversarial Networks comprises two sections, one section which generates the synthetic data and another section which is trained to discriminate if the generated data is a synthetic one or a real one, in the sense defined above.

In accordance to the present embodiment the generator generates a registered ultrasound image without having carried out any registration algorithm based on transfer functions calculated for example by using landmarks in two images to be registered one to the other. The discriminator analyzes the said synthetic registered image in order to discriminate whether this image is a synthetic one or a real one. In this case real meaning a registered image obtained by using transfer functions of the image pixels calculated basing on landmarks on the images to be registered one with respect to the other.

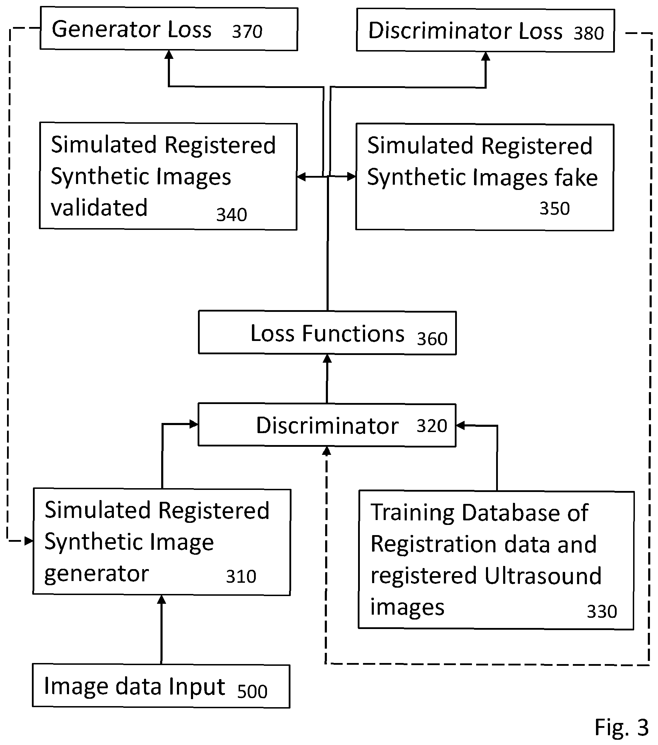

Figure 3 is a generic example of an embodiment of a Generative Adversarial Network for carrying out the calculation of the registration data according to the present disclosure.

Figure 3 shows a high-level diagram of a generic GANs algorithm. The said algorithm combines two neural networks or to groups of neural networks operating in competition one to the other. A first neural network having the function of the generator is indicated with 310 and has the function of generating simulated registered Synthetic Image data starting from the image data input 300 of the image data relating to the panoramic image acquired by the said high-depth and large FOV ultrasound scan. The said registered synthetic image data is fed to a discriminator 320, which is the further neural network or the further group of neural networks, which is trained to determine if the inputted image data can be considered real or fake. This means if the simulated registered image data can be held as registered image data obtained by applying to the pixels of an input image to be registered with another image transfer functions of the pixels of the said input image calculated by considering position and shape of landmarks which are present both in the input image and in the image with respect to which said input image must be registered.

The discriminator 320 is trained to carry out the above discrimination by a Training Database of digital images acquired by providing registration data in the target ROI (region of interest) which registration data corresponds to the transfer functions calculated as a functions of the position and shape of said one or more landmarks present in the image to be registered and in the reference image. And which database is indicated with 330.

The image data considered real by the discriminator will be the output of the validated Simulated registered Synthetic Image data 340. The image data classified as fake by the discriminator will be discarded and not considered.

Determining if the output images are real and fake is matter of the discriminator and loss functions are provided in order to calculate the quality of the output as indicated by 360. The loss functions are used in rewarding or punishing the generator 310 and the discriminator 320 respectively in the case that a generated synthetic registered image by the generator 310 is labelled as true by the discriminator 320 or if the discriminator recognized that a generated synthetic registered image by the generator 310 is not a real registered image but a synthetic one.

This is represented by the Generator loss and Discriminator loss functions which are indicated by 370 and 380. The result of the loss either causing reward or punishment may be fed back respectively to the generator 310 and to the discriminator 320 for further training of both, namely generator and discriminator such as learning from their success and mistakes.

Such a backpropagation training by means of the computed losses by means of the loss functions 360 may or may not be provided and this is indicated by the dotted arrow connecting the loss to the respective generator and discriminator unit 510, 520.

The above disclosed architecture is a generic architecture of a GAN and several neural networks of different kind either alone or grouped with other neural networks may be used for constructing the generator 310 and the discriminator 320, as well as also several different loss functions may be provided in order to determine the losses which are fed back to the generator and to the discriminator for enhancing their effectiveness and fitness.

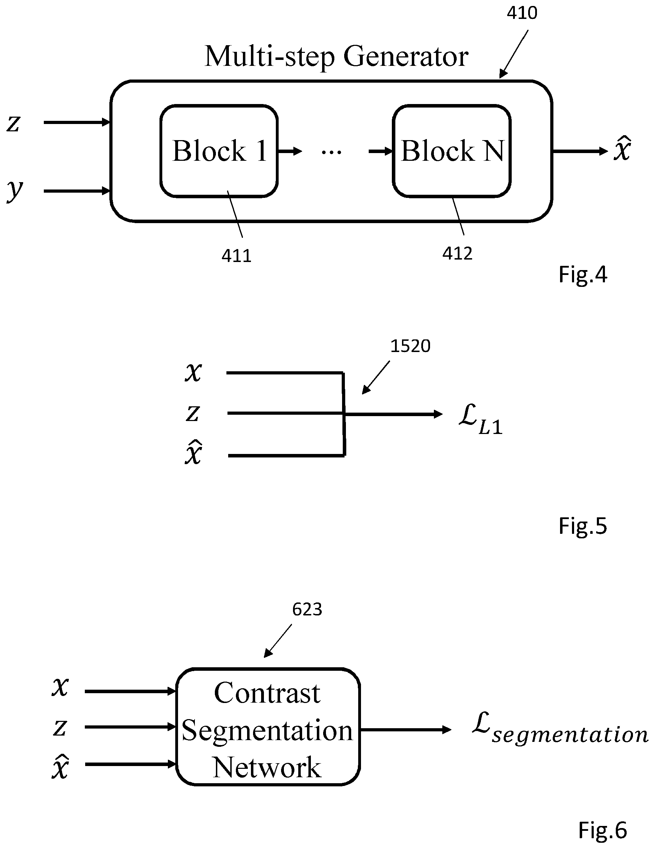

In the following some examples are disclosed for constructing a GAN starting from the generic architecture disclosed in figure 5.

Figure 4 shows an exemplary embodiment of a generator which is configured as a so-called Multistep generator. The said generator 410 is formed of multiple (N) blocks 411, 412 concatenated in an end-to-end manner. Each block is configured to progressively transform the input image data acquired without providing a chemical contrast agent indicated with (y) leading to a more realistic final output image being the synthetic contrast enhanced image generated and indicated by (*x̂*).

According to an embodiment the said block may be constructed each one as a neural network. The network consists of a contracting path and an expansive path, which gives it the u-shaped architecture. The contracting path is a typical convolutional network that consists of repeated application of convolutions, each followed by a rectified linear unit (ReLU) and a max pooling operation. During the contraction, the spatial information is reduced while feature information is increased. The expansive pathway combines the feature and spatial information through a sequence of up-convolutions and concatenations with high-resolution features from the contracting path. A more detailed description of the U-Net algorithm is disclosed in https://en.wikipedia.org/wiki/U-Net.

In the figure 5 and in the following figure 6 the following notation is used:
y: input pre-contrast MRI scan (2D image or 3D volume)
z: training label, namely 0: negative case 1: positive case
x: ground-truth post-registration of the ultrasound image scan (2D image or 3D volume), namely ultrasound images obtained by registration with the images acquired with the first imaging modality by using registration transfer functions;
*x̂*: output, namely the synthetic image generated starting from the non-registered ultrasound image by applying to it the registration data obtained from the above synthetic registration process and which provides a synthetic registered image which has to be fed to the discriminator for evaluation as true or fake.

The embodiment of figure 4 shows a schematic representation of a discriminator configured to operate with a traditional pixel wise loss function which is defined as 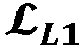 .

According to a feature of the present embodiment, this loss is used only to train the networks on negative cases. This is a concept of selective training consisting in losses being activated only under certain class-related conditions, particularly positive and negative cases for synthetic registration.

Still a further embodiment of the discriminator is shown in figure 6. The Discriminator 623 is defined as a Contrast Segmentation Network and the loss function as 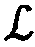 segmentation.

This network is pre-trained to segment contrast content within input images (x, *x̂*) . This network is used to segment both sets of input images (x, *x̂*) and penalize the differences between the resultant segmentation maps using the given segmentation loss function.

According to a further feature, this network is only activated for z=1, meaning positive images with high-contrast regions.

Other loss functions may be applied which are for example:
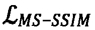 is a loss function penalizing the differences in multi-scale structural similarity index measure (SSIM) between the output and ground-truth images. More details are provided at https://en.wikipedia.org/wiki/Structural similarity;
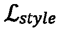 which is a loss function penalizing the differences in style, texture and details between the output and ground-truth images;
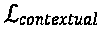 which is a state-of-the-art loss function used to match the localized semantic meaning of the input images while taking into account the entire global properties of the images and is used for the first time in medical image-to-image translation, particularly for breast MRI and Digital Contrast Agents (DCA) .

More detailed disclosure of Neural Style Transfer algorithms and the related loss functions can be found at https://en.wikipedia.org/wiki/Neural_Style_Transfer.

A detailed disclosure of the semantic loss can be found at https://shagunsodhani.com/papers-I-read/A-Semantic-Loss-Function-for-Deep-Learning-with-Symbolic-Knowledge.

Fig. 7 illustrates a block diagram of an ultrasound system formed in accordance with an alternative embodiment. The system of FIG. 7 implements the operations described herein in connection with various embodiments. By way of example, one or more circuits/processors within the system implement the operations of any processes illustrated in connection with the figures and/or described herein.

In particular any processor may be provided in combination with a software which comprises the instructions for carrying out at least one part of the functions and steps of the present method and which software is loaded and executed by the said processor/processors according to the embodiment already disclosed with reference to figure 1, in which the system for calculating the registration data and for generating the combination image is integrated in relation to the hardware in the hardware of the ultrasound system.

The system includes a probe interconnect board 702 that includes one or more probe connection ports 704. The connection ports 704 may support various numbers of signal channels (e.g., 128, 192, 256, etc.). The connector ports 704 may be configured to be used with different types of probe arrays (e.g., phased array, linear array, curved array, 1D, 1.25D, 1.5D, 1.75D, 2D array, etc.). The probes may be configured for different types of applications, such as abdominal, cardiac, maternity, gynecological, urological and cerebrovascular examination, breast examination and the like.

One or more of the connection ports 704 may support acquisition of 2D image data and/or one or more of the connection ports 704 may support 3D image data. By way of example only, the 3D image data may be acquired through physical movement (e.g., mechanically sweeping or physician movement) of the probe and/or by a probe that electrically or mechanically steers the transducer array.

The probe interconnect board (PIB) 702 includes a switching circuit 706 to select between the connection ports 704. The switching circuit 706 may be manually managed based on user inputs. For example, a user may designate a connection port 704 by selecting a button, switch or other input on the system. Optionally, the user may select a connection port 704 by entering a selection through a user interface on the system.

Optionally, the switching circuit 706 may automatically switch to one of the connection ports 704 in response to detecting a presence of a mating connection of a probe. For example, the switching circuit 706 may receive a "connect" signal indicating that a probe has been connected to a select one of the connection ports 704. The connect signal may be generated by the probe when power is initially supplied to the probe when coupled to the connection port 704. Additionally or alternatively, each connection port 704 may include a sensor 705 that detects when a mating connection on a cable of a probe has been interconnected with the corresponding connection port 704. The sensor 705 provides be ca connect signal to the switching circuit 706, and in response thereto, the switching circuit 706 couples the corresponding connection port 704 to PIB outputs 708. Optionally, the sensor 705 may be constructed as a circuit with contacts provided at the connection ports 704. The circuit remains open when no mating connected is joined to the corresponding connection port 704. The circuit is closed when the mating connector of a probe is joined to the connection port 704.

A control line 724 conveys control signals between the probe interconnection board 702 and a digital processing board 724. A power supply line 736 provides power from a power supply 740 to the various components of the system, including but not limited to, the probe interconnection board (PIB) 702, digital front end boards (DFB) 710, digital processing board (DPB) 726, the master processing board (M PB) 744, and a user interface control board (UI CB) 746. A temporary control bus 738 interconnects, and provides temporary control signals between, the power supply 740 and the boards 702, 710, 726, 744 and 746. The power supply 740 includes a cable to be coupled to an external AC power supply. Optionally, the power supply 740 may include one or more power storage devices (e.g. batteries) that provide power when the AC power supply is interrupted or disconnected. The power supply 740 includes a controller 742 that manages operation of the power supply 740 including operation of the storage devices.

Additionally or alternatively, the power supply 740 may include alternative power sources, such as solar panels and the like. One or more fans 743 are coupled to the power supply 740 and are managed by the controller 742 to be turned on and off based on operating parameters (e.g. temperature) of the various circuit boards and electronic components within the overall system (e.g. to prevent overheating of the various electronics).

The digital front-end boards 710 providing analog interface to and from probes connected to the probe interconnection board 702. The DFB 710 also provides pulse or control and drive signals, manages analog gains, includes analog to digital converters in connection with each receive channel, provides transmit beamforming management and receive beamforming management and vector composition (associated with focusing during receive operations).

The digital front end boards 710 include transmit driver circuits 712 that generate transmit signals that are passed over corresponding channels to the corresponding transducers in connection with ultrasound transmit firing operations. The transmit driver circuits 712 provide pulse or control for each drive signal and transmit beamforming management to steer firing operations to points of interest within the region of interest. By way of example, a separate transmit driver circuits 712 may be provided in connection with each individual channel, or a common transmit driver circuits 712 may be utilized to drive multiple channels. The transmit driver circuits 712 cooperate to focus transmit beams to one or more select points within the region of interest. The transmit driver circuits 712 may implement single line transmit, encoded firing sequences, multiline transmitter operations, generation of shear wave inducing ultrasound beams as well as other forms of ultrasound transmission techniques.

The digital front end boards 710 include receive beamformer circuits 714 that received echo/receive signals and perform various analog and digital processing thereon, as well as phase shifting, time delaying and other operations in connection with beamforming. The beam former circuits 714 may implement various types of beamforming, such as single-line acquisition, multiline acquisition as well as other ultrasound beamforming techniques.

The digital front end boards 716 include continuous wave Doppler processing circuits 716 configured to perform continuous wave Doppler processing upon received echo signals. Optionally, the continuous wave Doppler circuits 716 may also generate continuous wave Doppler transmit signals.

The digital front-end boards 710 are coupled to the digital processing board 726 through various buses and control lines, such as control lines 722, synchronization lines 720 and one or more data bus 718. The control lines 722 and synchronization lines 720 provide control information and data, as well as synchronization signals, to the transmit drive circuits 712, receive beamforming circuits 714 and continuous wave Doppler circuits 716. The data bus 718 conveys RF ultrasound data from the digital front-end boards 710 to the digital processing board 726. Optionally, the digital front end boards 710 may convert the RF ultrasound data to I,Q data pairs which are then passed to the digital processing board 726.

The digital processing board 726 includes an RF and imaging module 728, a color flow processing module 730, an RF processing and Doppler module 732 and a PCI link module 734. The digital processing board 726 performs RF filtering and processing, processing of black and white image information, processing in connection with color flow, Doppler mode processing (e.g. in connection with polls wise and continuous wave Doppler). The digital processing board 726 also provides image filtering (e.g. speckle reduction) and scanner timing control. The digital processing board 726 may include other modules based upon the ultrasound image processing functionality afforded by the system.

The modules 728 - 734 comprise one or more processors, DSPs, and/or FPGAs, and memory storing program instructions to direct the processors, DSPs, and/or FPGAs to perform various ultrasound image processing operations. The RF and imaging module 728 performs various ultrasound related imaging, such as B mode related image processing of the RF data. The RF processing and Doppler module 732 convert incoming RF data to I,Q data pairs, and performs Doppler related processing on the I, Q data pairs. Optionally, the imaging module 728 may perform B mode related image processing upon I, Q data pairs. The CFM processing module 730 performs color flow related image processing upon the ultrasound RF data and/or the I, Q data pairs. The PCI link 734 manages transfer of ultrasound data, control data and other information, over a PCI express bus 748, between the digital processing board 726 and the master processing board 744.

The master processing board 744 includes memory 750 (e.g. serial ATA solid-state devices, serial ATA hard disk drives, etc.), a VGA board 752 that includes one or more graphic processing unit (GPUs), one or more transceivers 760 one or more CPUs 752 and memory 754. The master processing board (also referred to as a PC board) provides user interface management, scan conversion and cine loop management. The master processing board 744 may be connected to one or more external devices, such as a DVD player 756, and one or more displays 758. The master processing board includes communications interfaces, such as one or more USB ports 762 and one or more ports 764 configured to be coupled to peripheral devices. The master processing board 744 is configured to maintain communication with various types of network devices 766 and various network servers 768, such as over wireless links through the transceiver 760 and/or through a network connection (e.g. via USB connector 762 and/or peripheral connector 764).

The network devices 766 may represent portable or desktop devices, such as smart phones, personal digital assistants, tablet devices, laptop computers, desktop computers, smart watches, ECG monitors, patient monitors, and the like. The master processing board 744 conveys ultrasound images, ultrasound data, patient data and other information and content to the network devices for presentation to the user. The master processing board 744 receives, from the network devices 766, inputs, requests, data entry and the like.

The network server 768 may represent part of a medical network, such as a hospital, a healthcare network, a third-party healthcare service provider, a medical equipment maintenance service, a medical equipment manufacturer, a government healthcare service and the like. The communications link to the network server 768 may be over the Internet, a private intranet, a local area network, a wide-area network, and the like.

The master processing board 744 is connected, via a communications link 770 with a user interface control board 746. The communications link 770 conveys data and information between the user interface and the master processing board 744. The user interface control board 746 includes one or more processors 772, one or more audio/video components 774 (e.g. speakers, a display, etc.). The user interface control board 746 is coupled to one or more user interface input/output devices, such as an LCD touch panel 776, a trackball 778, a keyboard 780 and the like. The processor 772 manages operation of the LCD touch panel 776, as well as collecting user inputs via the touch panel 776, trackball 778 and keyboard 780, where such user inputs are conveyed to the master processing board 744 in connection with implementing embodiments herein.

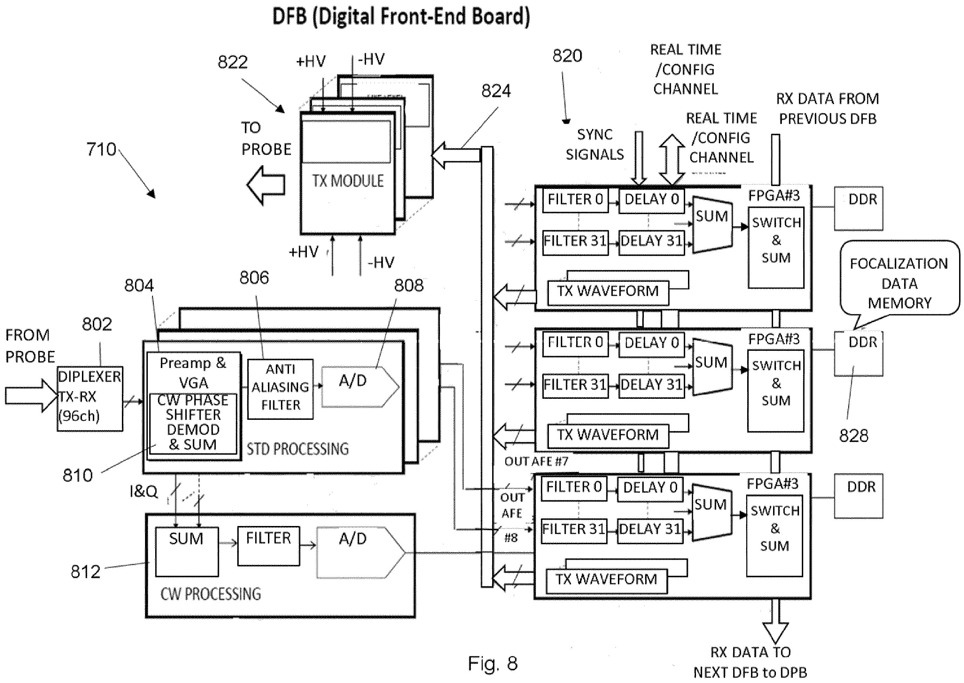

FIG. 8 illustrates a block diagram of a portion of the digital front-end boards 710 formed in accordance with embodiments herein. A group of diplexers 802 receive the ultrasound signals for the individual channels over the PIB output 808. The ultrasound signals are passed along a standard processing circuit 805 or to a continuous wave processing circuit 812, based upon the type of probing utilized. When processed by the standard processing circuit 805, a preamplifier and variable gain amplifier 804 process the incoming ultrasound receive signals that are then provided to an anti-aliasing filter 806 which performs anti-aliasing filtering. The output thereof is provided to an A/D converter 808 that digitizes the incoming analog ultrasound receive signals. When a continuous wave (CW) probe is utilized, the signals therefrom are provided to a continuous wave phase shifter, demodulator and summer 810 which converts the analog RF receive signals to I,Q data pairs. The CW I,Q data pairs are summed, filtered and digitized by a continuous wave processing circuit 812. Outputs from the standard or continuous wave processing circuits 805, 812 are then passed to beam forming circuits 820 which utilize one or more FPGAs to perform filtering, delaying and summing the incoming digitized receive signals before passing the RF data to the digital processing board 826 (FIG. 7). The FPGAs receive focalization data from memories 828. The focalization data is utilized to manage the filters, delays and summing operations performed by the FPGAs in connection with beamforming. The being formed RF data is passed between the beamforming circuits 820 and ultimately to the digital processing board 726.

The digital front-end boards 710 also include transmit modules 822 that provide transmit drive signals to corresponding transducers of the ultrasound probe. The beamforming circuits 820 include memory that stores transmit waveforms. The transmit modules 822 receive transmit waveforms over line 824 from the beamforming circuits 820.

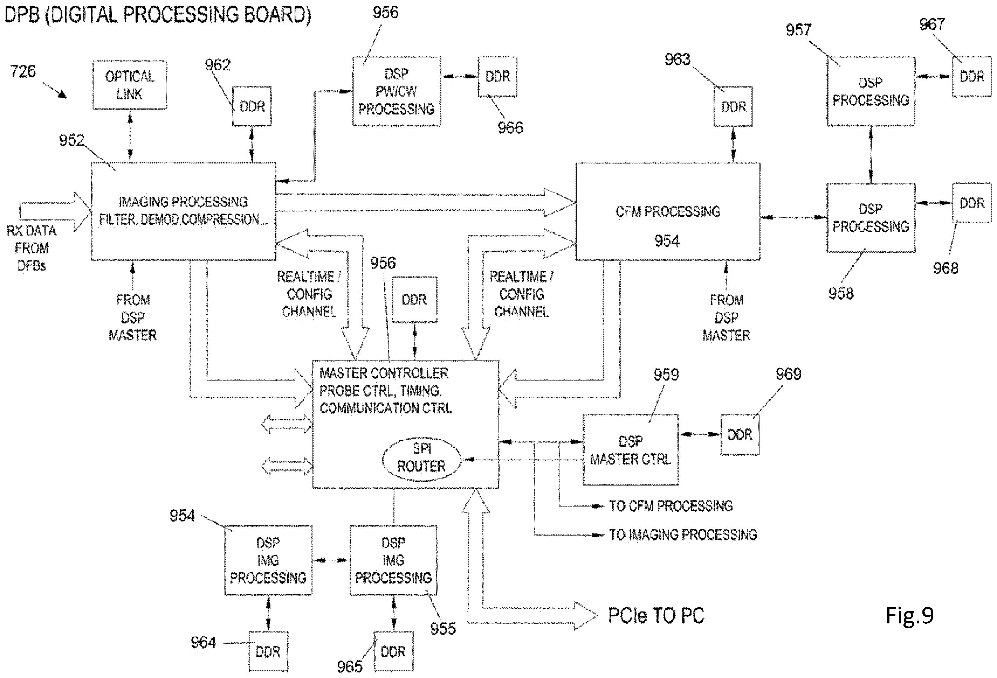

Fig. 9 illustrates a block diagram of the digital processing board 726 implemented in accordance with embodiments herein. The digital processing board 726 includes various processors 952-959 to perform different operations under the control of program instructions saved within corresponding memories see 962 - 969. A master controller 950 manages operation of the digital processing board 726 and the processors 952 - 959. By way of example, one or more processors as the 952 may perform filtering, the modulation, compression and other operations, while another processor 953 performs color flow processing. The master controller provides probe control signals, timing control signals, communications control and the like. The master controller 950 provides real-time configuration information and synchronization signals in connection with each channel to the digital front-end board 710.

Aspects are described herein with reference to the FIGS., which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

One or more of the operations described above in connection with the methods may be performed using one or more processors. The different devices in the systems described herein may represent one or more processors, and two or more of these devices may include at least one of the same processors. In one embodiment, the operations described herein may represent actions performed when one or more processors (e.g., of the devices described herein) execute program instructions stored in memory (for example, software stored on a tangible and non-transitory computer readable storage medium, such as a computer hard drive, ROM, RAM, or the like).

The processor(s) may execute a set of instructions that are stored in one or more storage elements, in order to process data. The storage elements may also store data or other information as desired or needed. The storage element may be in the form of an information source or a physical memory element within the controllers and the controller device. The set of instructions may include various commands that instruct the controllers and the controller device to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

The controller may include any processor-based or microprocessor-based system including systems using microcontrollers, reduced instruction set computers (RISC), application specific integrated circuitry (ASICs), field-programmable gate arrays (FPGAs), logic circuitry, and any other circuit or processor capable of executing the functions described herein. When processor-based, the controller executes program instructions stored in memory to perform the corresponding operations. Additionally or alternatively, the controllers and the controller device may represent circuitry that may be implemented as hardware. The above examples are exemplary only, and are thus not intended to limit in any way the definition and/or meaning of the term "controller."

Optionally, aspects of the processes described herein may be performed over one or more networks one a network server. The network may support communications using any of a variety of commercially-available protocols, such as Transmission Control Protocol/Internet Protocol ("TCP/IP"), User Datagram Protocol ("UDP"), protocols operating in various layers of the Open System Interconnection ("OSI") model, File Transfer Protocol ("FTP"), Universal Plug and Play ("UpnP"), Network File System ("NFS"), Common Internet File System ("CIFS") and AppleTalk. The network can be, for example, a local area network, a wide-area network, a virtual private network, the Internet, an intranet, an extranet, a public switched telephone network, an infrared network, a wireless network, a satellite network and any combination thereof.

In embodiments utilizing a web server, the web server can run any of a variety of server or mid-tier applications, including Hypertext Transfer Protocol ("HTTP") servers, FTP servers, Common Gateway Interface ("CGI") servers, data servers, Java servers, Apache servers and business application servers. The server(s) also may be capable of executing programs or scripts in response to requests from user devices, such as by executing one or more web applications that may be implemented as one or more scripts or programs written in any programming language, such as Java^{®}, C, C# or C++, or any scripting language, such as Ruby, PHP, Perl, Python or TCL, as well as combinations thereof. The server(s) may also include database servers, including without limitation those commercially available from Oracle^{®}, Microsoft^{®}, Sybase^{®} and IBM^{®} as well as open-source servers such as MySQL, Postgres, SQLite, MongoDB, and any other server capable of storing, retrieving and accessing structured or unstructured data. Database servers may include table-based servers, document-based servers, unstructured servers, relational servers, non-relational servers or combinations of these and/or other database servers.

The embodiments described herein may include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers or other network devices may be stored locally and/or remotely, as appropriate. Where a system includes computerized devices, each such device can include hardware elements that may be electrically coupled via a bus, the elements including, for example, at least one central processing unit ("CPU" or "processor"), at least one input device (e.g., a mouse, keyboard, controller, touch screen or keypad) and at least one output device (e.g., a display device, printer or speaker). Such a system may also include one or more storage devices, such as disk drives, optical storage devices and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.) and working memory as described above. The computer-readable storage media reader can be connected with, or configured to receive, a computer-readable storage medium, representing remote, local, fixed and/or removable storage devices as well as storage media for temporarily and/or more permanently containing, storing, transmitting and retrieving computer-readable information. The system and various devices also typically will include a number of software applications, modules, services or other elements located within at least one working memory device, including an operating system and application programs, such as a client application or web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Various embodiments may further include receiving, sending, or storing instructions and/or data implemented in accordance with the foregoing description upon a computer-readable medium. Storage media and computer readable media for containing code, or portions of code, can include any appropriate media known or used in the art, including storage media and communication media, such as, but not limited to, volatile and non-volatile, removable and non-removable media implemented in any method or technology for storage and/or transmission of information such as computer readable instructions, data structures, program modules or other data, including RAM, ROM, Electrically Erasable Programmable Read-Only Memory ("EEPROM"), flash memory or other memory technology, Compact Disc Read-Only Memory ("CD-ROM"), digital versatile disk (DVD) or other optical storage, magnetic cassettes, magnetic tape, magnetic disk storage or other magnetic storage devices or any other medium which can be used to store the desired information and which can be accessed by the system device. Based on the disclosure and teachings provided herein, a person of ordinary skill in the art will appreciate other ways and/or methods to implement the various embodiments.

The specification and drawings are, accordingly, to be regarded in an illustrative rather than a restrictive sense. It will, however, be evident that various modifications and changes may be made thereunto without departing from the scope of the invention as set forth in the claims.

Thus, while the disclosed techniques are susceptible to various modifications and alternative constructions, certain illustrated embodiments thereof are shown in the drawings and have been described above in detail. It should be understood, however, that there is no intention to limit the invention to the specific form or forms disclosed, but on the contrary, the intention is to cover all modifications, alternative constructions and equivalents falling within the scope of the invention, as defined in the appended claims.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including" and "containing" are to be construed as open-ended terms (i.e., meaning "including, but not limited to,") unless otherwise noted. The term "connected," when unmodified and referring to physical connections, is to be construed as partly or wholly contained within, attached to or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. The use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but the subset and the corresponding set may be equal.

Processes described herein (or variations and/or combinations thereof) may be performed under the control of one or more computer systems configured with executable instructions and may be implemented as code (e.g., executable instructions, one or more computer programs or one or more applications) executing collectively on one or more processors, by hardware or combinations thereof. The code may be stored on a computer-readable storage medium, for example, in the form of a computer program comprising a plurality of instructions executable by one or more processors. The computer-readable storage medium may be non-transitory.

Preferred embodiments of this disclosure are described herein, including the best mode known to the inventors for carrying out the invention. Variations of those preferred embodiments may become apparent to those of ordinary skill in the art upon reading the foregoing description. The inventors expect skilled artisans to employ such variations as appropriate and the inventors intend for embodiments of the present disclosure to be practiced otherwise than as specifically described herein. Accordingly, the scope of the present disclosure includes all modifications and equivalents of the subject matter recited in the claims appended hereto.

## Claims

1. Tracker-less, processor-implemented method for registering images acquired with different modalities for generating fusion images from registered images acquired with different modalities, the said method comprising the following steps:
- Acquiring ultrasound images by interlacing (220) a wide, high depth ultrasound scan (221) to a zoomed ultrasound scan (222);
- Registering (210) the image data obtained from the said high-depth ultrasound scan with image data of the same anatomical region acquired with a different modality and determining registration data (211) ;
- the said image data obtained from the said high-depth ultrasound scan and/or the said image data acquired with the different modality not being displayed to the user;
- Registering (230) the said image data acquired by the said zoomed ultrasound scan with corresponding zoomed image data obtained with the said different modality by applying the said registration data to the image data acquired by the said zoomed ultrasound scan ;
- Combining and/or fusing (240) the said registered image data acquired by the said zoomed ultrasound scan with the zoomed image data obtained with the said different modality and
- Displaying (250) the said combined or fused image data acquired by the said zoomed ultrasound scan with the zoomed image data obtained with the said different modality.

2. Method according to claim 1 in which registration is carried out by means of registration algorithms comprising the steps of:
- Defining landmarks on the image acquired by the different modality and by the wide, high-depth ultrasound scan;
- defining a spatial reference system common to both said images;
- determining transfer functions of the image pixels of the image according to the different modality to the image pixels of the image acquired by the high-depth ultrasound scan based on the different spatial positions of the said landmarks in the common reference system and in which the said transfer functions, also called registration data, are applied to the image pixels obtained by the zoomed ultrasound scan for registering the said image data acquired by the said zoomed ultrasound scan with the said different modality image and which registered zoomed ultrasound image is combined with a correspondingly zoomed field of view of the image acquired by the different modality and only the said combined image is displayed to the user.

3. Method according to claim 1 in which registration data is determined by using a so called generative algorithm as the so called GAN.

4. Method according to claim 3 in which a one-shot machine learning registration of heterogeneous imaging modalities is applied.

5. Method according to claim 3 in which a Machine Learning algorithm is used for mapping the image acquired by a different modality such as for example MRI or CT to a "synthetic" ultrasound image subsequent registration to a real ultrasound image.

6. Method according to claim 3 in which a Machine Learning algorithm is used for carrying out an anatomical segmentation in the ultrasound images and the registration with previously segmented images acquired by the other modality.

7. A system which is tracker-less and configured for registering images acquired with different modalities for generating fusion images from registered images acquired with different modalities, which system comprises:
- an ultrasound imaging system;
- a processing unit (112) configured to store (150) images acquired with a first imaging modality and images acquired by the ultrasound imaging system (117, 127);
- said processing unit being configured to calculate registration data (150) of an image acquired by the ultrasound system with an image acquired with the first modality;
- a zooming processor (140, 127) which sets the ultrasound imaging system for acquiring zoomed images;
- an image combination processor (160) which applies the registration data to a zoomed ultrasound image and combines the said zoomed ultrasound image with a corresponding zoomed field of view of the image acquired by the first imaging modality;
- a display (109) for displaying the combined zoomed ultrasound image with the said corresponding zoomed field of view of the image acquired by the first imaging modality and in which an ultrasound system controller (112, 106) is provided which is configured to drive the ultrasound system for carrying out in an interlaced manner a high-depth and large field of view imaging scan and a zoomed ultrasound scan, the said controller feeding the image data acquired by said high-depth and large field of view imaging scans to the said processing (150) for calculating the registration data with the image acquired by the first modality and not for being processed for display;
the said controller (112, 106) feeding the imaged data acquired by the zoomed ultrasound scan to the said processing unit for applying to it the registration data;
the said controller (112, 106) feeding the registered zoomed ultrasound image with the said corresponding zoomed field of view of the image acquired by the first imaging modality to the image combination processor (160) and the said combined image to the display (109).

8. A system according to claim 7, in which the processing unit as well as the image combination unit (160) can be in the form of a software coding the instructions for a processing unit (112, 106) to carry out the above disclosed functions.

9. A system according to claim 8 in which the said software is loaded and executed by a processing unit which is integrated or part of a CPU (112) of the ultrasound system.

10. A system according to claim 8 in which the said software is loaded and executed by an external CPU which is communicating with the controller (112, 106) of the ultrasound system and with the display (109) of the ultrasound system.

11. A system according to claim 8 in which part of the said software is loaded and executed by the processing unit, which is integrated, or is part of a CPU (112) of the ultrasound system and part of the software is loaded and executed by said external CPU.

## Patentansprüche

1. Trackerloses, prozessorimplementiertes Verfahren zur Registrierung von mit verschiedenen Modalitäten aufgenommenen Bildern zur Erzeugung von Fusionsbildern aus registrierten, mit verschiedenen Modalitäten aufgenommenen Bildern, wobei das Verfahren die folgenden Schritte umfasst:
- Aufnehmen von Ultraschallbildern durch Interlacing (220) eines breiten tiefreichenden Ultraschallscans (221) mit einem gezoomten Ultraschallscan (222);
- Registrieren (210) der aus dem besagten tiefreichenden Ultraschallscan aufgenommenen Bilddaten mit Bilddaten derselben anatomischen Region, die mit einer anderen Modalität aufgenommen wurden, und Bestimmen von Registrierungsdaten (211);
- wobei die genannten Bilddaten, die aus dem besagten tiefreichenden Ultraschallscan aufgenommen wurden, und/oder die besagten Bilddaten, die mit der anderen Modalität aufgenommen wurden, dem Benutzer nicht angezeigt werden;
- Registrieren (230) der durch den gezoomten Ultraschallscan aufgenommenen Bilddaten mit entsprechenden gezoomten Bilddaten, die mit der anderen Modalität durch Anwenden der Registrierungsdaten auf die durch den gezoomte Ultraschallscan erfassten Bilddaten aufgenommen wurden,
- Kombinieren und/oder Fusionieren (240) der durch den gezoomte Ultraschallscan aufgenommenen registrierten Bilddaten mit den mit der anderen Modalität erhaltenen gezoomten Bilddaten und
- Anzeigen (250) der durch den gezoomten Ultraschallscan aufgenommenen kombinierten oder fusionierten Bilddaten mit den mit der anderen Modalität erhaltenen gezoomten Bilddaten.

2. Verfahren nach Anspruch 1, wobei die Registrierung mittels Registrierungsalgorithmen durchgeführt wird, umfassend die folgenden Schritte:
- Definieren von Landmarken auf dem Bild, das durch unterschiedliche Modalitäten und durch den breiten tiefreichenden Ultraschallscan aufgenommenen wurde;
- Definieren eines räumlichen Referenzsystems, das beiden Bildern gemeinsam ist;
- Bestimmen von Transferfunktionen der Bildpixel des Bildes gemäß der unterschiedlichen Modalität zu den Bildpixeln des mittels tiefreichenden Ultraschallscan aufgenommenen Bildes aufgrund der unterschiedlichen Raumpositionen der besagten Landmarken im gemeinsamen Referenzsystem, wobei die Transferfunktionen, auch als Registrierungsdaten bezeichnet, auf die durch den gezoomten Ultraschallscan gewonnenen Bildpixel angewendet werden, um die durch den gezoomten Ultraschallscan aufgenommenen Bilddaten mit dem Bild mit verschiedener Modalität zu registrieren, und wobei das registrierte gezoomte Ultraschallbild mit einem entsprechend gezoomten Sichtfeld des durch die verschiedene Modalität aufgenommenen Bildes kombiniert wird und nur das kombinierte Bild dem Benutzer angezeigt wird.

3. Verfahren nach Anspruch 1, wobei die Registrierungsdaten unter Verwendung eines so genannten generativen Algorithmus wie dem so genannten GAN bestimmt werden.

4. Verfahren nach Anspruch 3, wobei eine Registrierung heterogener Bildgebungsmodalitäten mittels One-Shot-Maschinelles Lernen angewendet wird.

5. Verfahren nach Anspruch 3, wobei ein Algorithmus für maschinelles Lernen zum Mapping des mit einer verschiedenen Modalität, wie beispielsweise MRT oder CT, aufgenommenen Bildes auf ein "synthetisches" Ultraschallbild und zur anschließenden Registrierung mit einem realen Ultraschallbild verwendet wird.

6. Verfahren nach Anspruch 3, wobei ein Algorithmus für maschinelles Lernen zur Durchführung einer anatomischen Segmentierung in den Ultraschallbildern und zur Registrierung mit zuvor segmentierten, mit der weiteren Modalität aufgenommenen Bildern verwendet wird.

7. System, das trackerlos ist und für die Registrierung von mit verschiedenen Modalitäten aufgenommenen Bildern konfiguriert ist, um aus registrierten, mit verschiedenen Modalitäten aufgenommenen Bildern Fusionsbilder zu erzeugen, wobei das System umfasst:
- ein Ultraschallbildgebungssystem;
- eine Verarbeitungseinheit (112), die zum Speichern (150) von mit einer ersten Bildgebungsmodalität aufgenommenen Bildern und von dem Ultraschallbildgebungssystem (117,127) aufgenommenen Bildern konfiguriert ist;
- wobei die Verarbeitungseinheit so konfiguriert ist, dass sie Registrierungsdaten (150) eines vom Ultraschallsystem erfassten Bildes mit einem mit der ersten Modalität erfassten Bild berechnet;
- einen Zoomprozessor (140, 127), der das Ultraschallbildgebungssystem zur Erfassung von gezoomten Bildern einstellt;
- einen Bildkombinationsprozessor (160), der die Registrierungsdaten auf ein gezoomtes Ultraschallbild anwendet und das gezoomte Ultraschallbild mit einem entsprechenden gezoomten Sichtfeld des durch die erste Bildgebungsmodalität aufgenommenen Bildes kombiniert;
- eine Anzeige (109) zum Anzeigen des kombinierten gezoomten Ultraschallbildes mit dem entsprechenden gezoomten Sichtfeld des durch die erste Bildgebungsmodalität aufgenommenen Bildes, wobei eine Ultraschallsystemsteuerung (112, 106) vorgesehen ist, die so konfiguriert ist, das Ultraschallsystem zum Ausführen im Interlaced-Modus eines tiefreichenden Bildgebungsscans mit breitem Sichtfeld und eines gezoomten Ultraschallscans zu steuern, wobei die Steuerung die durch den tiefreichenden Bildgebungsscan mit breitem Sichtfeld aufgenommenen Bilddaten an die besagte Verarbeitung (150) zur Berechnung der Registrierungsdaten mit dem durch die erste Modalität aufgenommenen Bild und nicht zur Verarbeitung für Anzeigezwecke zuführt;
die besagte Steuereinheit (112, 106) die durch den gezoomten Ultraschallscan gewonnenen Bilddaten an die besagte Verarbeitungseinheit zuführt, um auf diese die Registrierungsdaten anzuwenden;
die besagte Steuereinheit (112, 106) das registrierte gezoomte Ultraschallbild mit dem besagten entsprechenden gezoomten Sichtfeld des durch die erste Bildgebungsmodalität gewonnenen Bildes an den Bildkombinationsprozessor (160) und das besagte kombinierte Bild an die Anzeige (109) zuführt.

8. System gemäß Anspruch 7, wobei sowohl die Verarbeitungseinheit als auch die Bildkombinationseinheit (160) in Form einer Software vorliegen können, die die Anweisungen für eine Verarbeitungseinheit (112, 106) zur Ausführung der oben beschriebenen Funktionen codiert.

9. System gemäß Anspruch 8, wobei die Software von einer Verarbeitungseinheit geladen und ausgeführt wird, die in eine CPU (112) des Ultraschallsystems integriert oder Teil davon ist.

10. System gemäß Anspruch 8, wobei die Software von einer externen CPU geladen und ausgeführt wird, die mit der Steuereinheit (112, 106) des Ultraschallsystems und mit der Anzeige (109) des Ultraschallsystems kommuniziert.

11. System gemäß Anspruch 8, wobei ein Teil der besagten Software von der Verarbeitungseinheit geladen und ausgeführt wird, die integriert ist oder Teil einer CPU (112) des Ultraschallsystems ist, und ein Teil der Software von der besagten externen CPU geladen und ausgeführt wird.

## Revendications

1. Méthode sans traceur, mis en œuvre par processeur, pour enregistrer des images acquises avec modalités différentes afin de générer des images fusionnées à partir d'images enregistrées acquises avec modalités différentes, ladite méthode comprenant les étapes suivantes:
- Acquérir des images ultrasonores en entrelaçant (220) un balayage ultrasonore large et à grande profondeur (221) avec un balayage ultrasonore agrandi (222);
- Enregistrer (210) les données d'image obtenues à partir dudit balayage ultrasonore à grande profondeur avec des données d'images de la même région anatomique acquises selon une modalité différente et déterminer des données d'enregistrement (211);
- lesdites données d'image obtenues à partir dudit balayage ultrasonore à grande profondeur et/ou lesdites données d'image acquises avec la modalité différente n'étant pas affichées à l'utilisateur;
- Enregistrer (230) lesdites données d'image acquises par ledit balayage ultrasonore agrandi avec des données d'image agrandies correspondantes obtenues avec ladite modalité différente en appliquant lesdites données d'enregistrement aux données d'image acquises par ledit balayage ultrasonore agrandi;
- Combiner et/ou fusionner (240) lesdites données d'image enregistrées acquises par ledit balayage ultrasonore agrandi avec les données d'image agrandies obtenues avec ladite modalité différente et
- Afficher (250) lesdites données d'image combinées ou fusionnées acquises par ledit balayage ultrasonore agrandi avec les données d'image agrandies obtenues par ladite modalité différente.

2. Méthode selon la revendication 1, où l'enregistrement est effectué au moyen d'algorithmes d'enregistrement comprenant les étapes de:
- Définir des points de repère sur l'image acquise par la modalité différente et par balayage ultrasonore large et à grande profondeur;
- définir un système de référence spatiale commun auxdites deux images;
- déterminer les fonctions de transfert des pixels d'image de l'image selon la modalité différente vers les pixels d'image de l'image acquise par le balayage ultrasonore à haute profondeur sur la base des positions spatiales différentes desdits points de repère dans le système de référence commun et où lesdites fonctions de transfert, également appelées données d'enregistrement, sont appliquées aux pixels d'image obtenus par le balayage ultrasonore agrandi pour enregistrer lesdites données d'image acquises par ledit balayage ultrasonore agrandi avec ladite image de modalité différente et laquelle image ultrasonore agrandie enregistrée est combinée avec un champ de vision agrandi de manière correspondante de l'image acquise par la modalité différente et seule ladite image combinée est affichée à l'utilisateur.

3. Méthode selon la revendication 1, où les données d'enregistrement sont déterminées à l'aide d'un algorithme dit génératif tel que ce qui est appelé GAN.

4. Méthode selon la revendication 3, où est appliqué un enregistrement d'apprentissage automatique de type one-shot de modalités d'imagerie hétérogènes.

5. Méthode selon la revendication 3, où un algorithme d'apprentissage automatique est utilisé pour mettre en correspondance l'image acquise par une modalité différente, telle que IRM ou TDM, avec une image ultrasonore « synthétique » après enregistrement sur une image ultrasonore réelle.

6. Méthode selon la revendication 3, où un algorithme d'apprentissage automatique est utilisé pour effectuer une segmentation anatomique dans les images ultrasonores et l'enregistrement avec des images précédemment segmentées acquises par l'autre modalité.

7. Un système sans traceur, configuré pour enregistrer des images acquises avec modalités différentes afin de générer des images fusionnées à partir d'images enregistrées acquises avec modalités différentes, lequel système comprend:
- un système d'imagerie par ultrasons;
- une unité de traitement (112) configurée pour stocker (150) des images acquises avec une première modalité d'imagerie et des images acquises par le système d'imagerie par ultrasons (117, 127);
- ladite unité de traitement étant configurée pour calculer des données d'enregistrement (150) d'une image acquise par le système à ultrasons avec une image acquise avec la première modalité;
- un processeur d'agrandissement (140, 127) qui règle le système d'imagerie par ultrasons pour acquérir des images agrandies;
- un processeur (160) de combinaison d'images qui applique les données d'enregistrement à une image ultrasonore agrandie et combine ladite image ultrasonore agrandie avec un champ de vision agrandi correspondant de l'image acquise par la première modalité d'imagerie;
- un écran (109) pour afficher l'image ultrasonore agrandie combinée avec ledit champ de vision agrandi correspondant de l'image acquise par la première modalité d'imagerie et dans lequel est prévu un contrôleur (112, 106) de système à ultrasons, qui est configuré pour commander le système à ultrasons afin d'effectuer de manière entrelacée un balayage d'imagerie à grande profondeur et à grand champ de vision et un balayage ultrasonore agrandi, ledit contrôleur fournissant audit traitement (150) les données d'image acquises par lesdits balayages d'imagerie à grande profondeur et à grand champ de vision pour calculer les données d'enregistrement avec l'image acquise par la première modalité et non pour être traitées en vue d'un affichage;
ledit contrôleur (112, 106) fournissant à ladite unité de traitement les données d'image acquises par le balayage ultrasonore agrandi, afin d'y appliquer les données d'enregistrement;
ledit contrôleur (112, 106) fournissant l'image ultrasonore agrandie enregistrée avec ledit champ de vision agrandi correspondant de l'image acquise par la première modalité d'imagerie, au processeur de combinaison d'images (160) et ladite image combinée à l'écran (109).

8. Un système selon la revendication 7, où l'unité de traitement ainsi que l'unité de combinaison d'images (160) peuvent se présenter sous la forme d'un logiciel codant les instructions destinées à une unité de traitement (112, 106) pour exécuter les fonctions décrites ci-dessus.

9. Un système selon la revendication 8, où ledit logiciel est chargé et exécuté par une unité de traitement qui est intégrée ou fait partie d'une UCC (112) du système à ultrasons.

10. Un système selon la revendication 8, où ledit logiciel est chargé et exécuté par une UCC externe qui communique avec le contrôleur (112, 106) du système à ultrasons et avec l'écran (109) du système à ultrasons.

11. Un système selon la revendication 8, où une partie dudit logiciel est chargée et exécutée par l'unité de traitement, qui est intégrée ou fait partie d'une UCC (112) du système à ultrasons, et une partie du logiciel est chargée et exécutée par ladite UCC externe.
